# EUROPEAN PATENT SPECIFICATION

(11) **EP 1 581 195 B1**
(45) Date of publication and mention of the grant of the patent: **26.05.2010**
(21) Application number: 03795890.7
(22) Date of filing: 12.12.2003
(51) Int. Cl.: A61K 9/28, A61K 36/00

(54) **FILM COATED TABLET COMPRISING AN EXTRACT OF RED VINE LEAVES**
FILMTABLETTE MIT EXTRAKT AUS ROTEN WEINBLÄTTERN
COMPRIMES ENROBES PAR UN FILM COMPRENANT UN EXTRAIT DE FEUILLES DE VIGNE ROUGES

(30) Priority: 31.12.2002 EP 02029108; 05.09.2003 EP 03019636
(43) Date of publication of application: 05.10.2005
(73) Proprietor: Boehringer Ingelheim International GmbH, 55216 Ingelheim am Rhein (DE)
(72) Inventor: ESPERESTER, Anke, 55126 Mainz (DE); SCHAEFER, Eckhard, 63150 Heusenstamm (DE); SACHER, Fritz, 55435 Gau-Algesheim (DE)
(86) International application number: PCT/EP2003/014148
(87) International publication number: WO 2004/058227

(56) References cited:
- WO-A-01/28363
- WO-A-02/072118
- US-A- 4 897 270
- US-B1- 6 297 218
- US-B1- 6 485 727

## Description

### BACKGROUND OF THE INVENTION

### 1. TECHNICAL FIELD

The invention relates to a film coated tablet comprising a dried extract of red vine leaves, an excipient and a tablet film and the use thereof for the improvement of the blood circulation and/or the oxygen supply of the lower extremities.

### 2. BACKGROUND INFORMATION

Chronic venous insufficiency (CVI) is a progredient disease and will lead in many patients - especially if untreated - to oedema, coronal phlebectasia (Widmer stage I), hyperpigmentation, induration, lipodermatosclerosis, white atrophy (Widmer stage II), or varicose leg ulcers (Widmer stage III). Chronically disturbed haemodynamics of deep or superficial veins due to obstructed venous segments or valvular incompetence lead usually to skin diseases in the inner ankle area of the lower limbs.1 Disturbances in the microcirculation of the skin have been considered to be major contributors for skin changes associated with chronic venous hypervolaemia and venous hypertension. (e.g. Fagrell B Vital microscopy and the pathophysiology of deep venous insufficiency. Int Angiol 1995;14:18-22.; Jünger M, Klyscz T, Hahn M, Rassner G. Disturbed blood flow regulation in venous leg ulcers. Int J Microcirc 1996;16:259-265).

Obviously, cutaneous microangiopathy of clinical relevance such as enlarged, tortuous capillaries surrounded by micro-oedema contributes to the skin alterations in the lower limbs and determines the course of CVI (Fagrell B, *loc. cit.* and Jünger M et al., *loc. cit*.).

The application of the laser Doppler technique in venous disorders is well illustrated. (e.g. Tulevski II, Ubbink DT, Jacobs MJHM. Red and green laser Doppler compared with capillary microscopy to assess skin microcirculation in the feet of healthy subjects. Microvasc Res 1999;58(2):83-88 and Bollinger A, Jäger K, Jünger M, Seifert H. The vascular laboratory: advances in non-invasive techniques. World J Surg 1988; 12:724-731).

Different techniques have been developed to investigate microcirculation in both functionally different layers of the skin: the deeper, mainly thermoregulatory layer and the superficial, nutritive layer. Microcirculatory disturbances in the superficial nutritive layer are of utmost relevance for trophical skin changes. (Jünger M et al., *loc*. *cit*. and Gschwandmer ME, Ambrozy B, Pasching S, Willfort A, Schneider B, Böhler K, et al. Microcirculation in venous ulcers and surrounding skin: findings with capillary microscopy and laser Doppler imager. Eur J Clin Invest 1999;29:708-716).

US 6 297 218 discloses phospholipid complex extracts of vitis vinifera. WO 02/072118 discloses aqueous organic decoctions for treating

The British patent GB 934,554 discloses that the capillary resistance of guniea pigs deficient in a vitamin can be enhanced by intraperitonally administration of an alcoholic extract of vine leaves.

The International patent application WO 01/28363 and its equivalent US 6,485,727 discloses a method for preventing or alleviating the discomfort associated with mild-to-moderate chronic venous insufficiency of the lower extremities with the aid of an aqueous extract of red vine leaves. In addition a daily dosage regimen of 80 to 1000 mg divided up in 1 to 3 capsules is suggested.

The problem underlying the present invention was to provide a dosage form which allows to administer such high amounts of aqueous extract of red vine leaves in the suggested regimen. In view of patient compliance there is the requirement that such dosage forms shall not be too big, in order to facilitate swallowing. On the other hand the dosage form must have a high stability to ensure long shelf storage times. Moreover, high bioavailability is but a prerequisite for the therapeutic and/or preventive success of such a dosage form.

### BRIEF SUMMARY OF THE INVENTION

It has been surprisingly found that a film coated tablet according to claim 1 fulfils these requirements and can be used to significantly enhance the microcirculation and the oxygen supply at the predominantly affected perimalleolar area of the leg in CVI patients.

Another aspect of the present invention is a process for preparing such a film coated tablet comprising the steps of:
(A) mixing the dried aqueous extract of red vine leaves (a) with the excipients (b), optionally in the presence of a volatile diluent;
(B) optionally screening the mixture obtained;
(C) compressing the mixture with a suitable tablet press; and
(D) coating the resulting tablet with the tablet film (c).

Furthermore, the invention relates the use of such a film coated tablet for preparing a pharmaceutical or dietary composition for the treatment or prevention of the discomfort, disorder and/or disease associated with chronic venous hypervolaemia and venous hypertension.

Furthermore, the invention relates to an aqueous extract of red vine leaves, which is obtainable by a method comprising the steps of:
(a) collecting red vine leaves at a point of time when the content in flavonoids has reached an optimum;.
(b) drying and crushing the leaves;
(c) cutting the leaves to pieces;
(d) extracting the leaves with water at elevated temperatures for 6 to 10 hours;
(e) concentrating and drying the obtained extract, and
(f) addition of up to 10 % by weight of silica relating to the final total amount of the resulting extract during the drying process.

### BRIEF DESCRIPTION OF THE DRAWINGS

Figure 1 shows the schematic design of the clinical study which has been carried out to prove the efficacy of the film coated tablet according to the present invention.
Figure 2 shows the influence of the vine leaf extract containing film coated tablet
   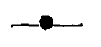 AS195 360 mg compared with
   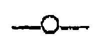 placebo
      on the microcirculation measured with Laser Doppler flowmetry (LDF 10-37 kHz).
Figure 3 shows the influence of the vine leaf extract containing film coated tablet
   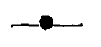 AS195 360 mg compared with
   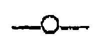 placebo
   on Transcutaneous oxygen partial pressure (tpO₂)

### DETAILED DESCRIPTION OF THE INVENTION

The film coated tablet of the present invention consists of herbal ingredients derived by an aqueous extraction from red vine leaves (*folia vitis viniferae*; Extractum Vitis viniferae e folium spissum et siccum) and drying (a), excipients (b) and the tablet film (c). This extract contains flavon(ol)-glycosides, -glucuronides and flavonoids, with quercetin-3-O-β-D-glucuronide and isoquercitrin (quercetin-3-O-β-glucoside) as its main active ingredients. The range of their pharmacological actions has not yet been fully elucidated, but in-vitro studies indicate that they have antioxidant and anti-inflammatory properties and that they inhibit platelet aggregation and hyaluronidase and reduce oedema, possibly by reducing capillary permeability, Preclinical in-vivo experiments demonstrated anti-inflammatory and capillary wall thickening effects

The film coated tablet according to the present invention comprises 50 to 70% of a dried aqueous red vine leaf extract with a high flavonoid content of 2-15%.

As a rule the relation by weight between the dried extract and the excipients used to produce the core of the tablet is between 1 : 1 to 2 : 1, preferably between 1.1 : 1 and 1.8 : 1, in particular between 1.25 :1 and 1.75 : 1.

A film coated tablet comprising
(a) 50 to 70 % by weight of said dried extract of red vine leaves;
(b) 25 to 49 % by weight of said excipient, and
(c) 1 to 5 % by weight of said tablet film,
based on the total mass of the film coated tablet, is preferred, wherein the dried as means extract of red vine leaves is obtainable by a drying process comprising the step of adding Fitich during the drying process.

More preferred is a film coated tablet comprising
(a) 51 to 59 %, in particular about 55 % by weight of said dried extract of red vine leaves;
(b) 38 to 48 %, in particular about 43 % by weight of said excipient, and
(c) 1 to 3 %, in particular about 2.7 % by weight of said tablet film,
based on the total of the film coated tablet wherein the dried aqueous extract of red vine leaves is obtainable by a drying process comprising the step of adding silica during the drying process.

Another preferred embodiment is a film coated tablet according, wherein the excipient (b) essentially consists of
- 70 to 85 % by weight of at least one binder,
- 0,5 to 12,5 % by weight of at least one disintegrant,
- 5 to 15 % by weight of at least one filler, and
- 1 to 5 % by weight of at least one lubricant,
based on the total mass of the combined excipients.

The term "binder" a used hereinbefore and hereinafter denotes an excipient which is suitable for binding other components to one another. Preferred binders according to the invention are selected from among:
powdered cellulose, microcrystalline cellulose, sorbitol, starch, polyvinylpyrrolidone. (povidone), copolymers of vinylpyrrolidone with other vinyl derivatives (copovidone), cellulose derivatives, particularly methylhydroxypropylcellulose, e.g. Methocel A 15 LV, and mixtures of these compounds. The preferred binders are powdered cellulose, particularly micracrystalline cellulose and/or copovidone. If the abovementioned binders are used, the amount by weight, based on the total mass of the tablet according to the invention, is preferably in a range of 15 - 45 wt.%, more preferably 25 - 40 wt.%, most preferably about 33 wt.%. Thanks to the particularly preferred binder microcrystalline cellulose, tablets are obtained having a high stability and good compliance for the patients to whom the aqueous extract of red vine leaves has to be aministered.

The tablet according to the invention also contains disintegrants in addition to the abovementioned ingredients. Within the scope of the present invention these disintegrants may optionally also be known as breakdown agents. These are preferably selected, according to the invention, from among sodium starch glycolate, crosslinked polyvinylpyrrolidone (crospovidone), croscarmellose sodium salt (sodium salt of cellulose carboxymethyl ether, crosslinked), sodium-carboxymethylcellulose, dried maize starch, colloidal anhydrous silica and mixtures thereof. Within the scope of the present invention it is particularly preferred to use sodium starch glycolate, crospovidone and, preferably, the sodium salt of crospovidone or croscarmellose and colloidal anhydrous silica. Most preferred is a mixture of croscarmellose sodium, colloidal anhydrous silica and optionally crospovidone. If the abovementioned disintegrants are used, the amount by weight, based on the total mass of the tablet according to the invention, is preferably in a range of about 0.5 -10 wt.%, more preferably about 1.5 - 7.5 wt.%. Thanks to the particularly preferred combination of disintegrants, tablets are obtained having a high stability and provide high bioavailability to the aqueous extract of red vine leaves.

The tablet according to the invention also contains a filler. As a rule fillers are inert compounds such as inorganic metal oxides or inorganic phosphate or hydrogen phosphate. Preferably the filler is anhydrous calcium hydrogen phosphate. If the abovementioned fillers are used, the amount by weight, based on the total mass of the tablet according to the invention, is preferably in a range of about 1 - 10 wt.%, more preferably about 2 - 8 wt.%.

The tablet according to the invention also contains flow agents or flow regulators and also lubricants, as additional ingredients. These include, within the scope of the present invention, for example, silicon dioxide, talc, stearic acid, sodium, stearylfumarate, magnesium stearate and glycerol tribehenate. According to the invention magnesium stearate is preferably used. If the abovementioned preferred lubricants are used, the amount by weight thereof, based on the total mass of the tablet according to the invention, is preferably in a range of about 0.1 - 10 wt.%, preferably about 0.5 - 5 wt.%, more preferably between 0.6 and 1.5 wt.%.

Furthermore preferred is a film coated tablet, wherein the tablet film (c) essentially consists of
- 50 to 85 % by weight of at least one film former,
- 5 to 10 % by weight of at least one plasticizer,
- 10 to 20 % by weight of at least one coating agent such as talc, and
- 0 to 15 % by weight of at least one colorant.
based on the total mass of the tablet film (c).

The tablet film according to the invention may also contain one or more synthetic or natural, pharmaceutically acceptable colorant, preferably one or more inorganic metal oxides such as titanium dioxide (E 171) and/or ferric oxide (E 172). If the abovementioned preferred colorants are used the amount by weight thereof based on the total mass of the tablet according to the invention is 0.01 to 0.5 wt.%.

It is a further object of the present invention to provide a film coated tablet for preventing and/or alleviating the discomfort associated with mild-to-moderate chronic venous insufficiency of the lower extremities comprising herbal ingredients, wherein the tablet is manufactured pursuant to a controlled process that preserves the herbal curing qualities of the ingredients.

It is still a further object of the present invention to provide a film coated tablet which is effective in preventing and/or alleviating the discomfort associated with mild-to-moderate chronic venous insufficiency of the lower extremities.

It is still a further object of the present invention to provide a film coated tablet for preventing and/or alleviating the discomfort associated with mild-to-moderate chronic venous insufficiency of the lower extremities comprising herbal ingredients and having minimal or no side effects and thus being safe for internal consumption and a high stability and good patient compliance.

The aqueous extract prepared from dried red vine leaves is characterised by a high content of 2 to 20%, preferably 2 to 10 % of biologically active flavonoids.

The term "a person in need thereof" or "patient" as used hereinabove and hereinbelow relates to a female or male person who suffers from clinically not relevant early stages of chronic venous insufficiency (CVI) from proven CVI stage I and II according to Widmer. As a rule such patients are elderly people with an age of between 30 and 80, preferably between 32 and 76 years having an mean age (± standard deviation) of 55.2 ± 7.7 years. As a rule CVI is more expressed in female than in male patients.

In order that this invention be more fully understood, the following examples are set forth. These examples are for the purpose of illustrating embodiments of this invention, and are not to be construed as limiting the scope of the invention in any way.

The examples which follow are illustrative and, as recognised by one skilled in the art, particular conditions could be modified as needed for individual compositions. Materials used in tests below are either commercially available or easily prepared from commercially available materials by those skilled in the art.

The basis of the tablet is the aqueous extract of red vine leaves (*foliae vitis viniferae L*.). The starting material for the preparation of the extract are red vine leaves collected at a point of time where the content in flavonoids has reached an optimum. This is usually the case around the harvesting time of the grapes. The leaves are carefully dried and crushed. For extraction the leaves are cut to pieces of preferably 5 to 10 mm. To achieve a high content in flavonoids the extraction is done at elevated temperature, preferably at a temperature in the range of 60° to 80°C, over a time of at least 6 up to 10 hours. The preferred method is that of an exhaustive percolation.

The so-called fluid extract obtained in the course of the extraction is concentrated by use of a suitable evaporator. The thick extract obtained in this step is dried, for instance by use of a vacuum drying oven or a vacuum drying conveyer.

All or some of the excipients may be added during drying to facilitate further processing of the extract. As a rule up to 10 % of one or more constituents of the excipients can be added during the drying process.

Preferably a part of the flow regulator such as colloidal, anhydrous silica is added to the extract during drying or before admixing with the other constituents. Preferably the resulting extract composition contains 0.5 to 10 % by weight, in particular 2.5 to 7.5 % by weight, most preferably about 4 % by weight of colloidal, anhydrous silica.

Surprisingly, tablets obtained from an extract to which a part of the excipients have been added during the drying process show an enhanced stability.

Most preferably the film coated tablet according to this invention consists of
■ 300 to 500 mg, preferably 320 to 400 mg, in particular about 355 to 380 mg of dry aqueous extract of red vine leaf (4-6 : 1) (*extractum vitis viniferae foliae aquosum siccum*), which may contain up to 10 % weight of a flow regulator, in particular colloidal, anhydrous silica;
■ the following excipients of the tablet core:
   microcrystalline cellulose, croscarmellose sodium, calcium hydrogen phosphate (anhydrous), colloidal silica (anhydrous), magnesium stearate, and optionally crospovidone, and
■ a tablet film consisting of:
   hypromellose, glyceryl tristearate, titanium dioxide (E 171), talc, ferric oxide, red (E 172).

Film coated tablets were prepared with the ingredients listed in the following tables A and B:

**Table A**

| Name of ingredient | Quantity per film coated tablet [mg / 658.000 mg] | Function |
|---|---|---|
| Tablet Core | | |
| Vitis viniferae folium dry extract aqueous (4 - 6 : 1) | 360.000 | Active ingredient |
| Microcrystalline cellulose | 219.000 | Binder, disintegrant |
| Croscarmellose sodium | 18.000 | Disintegrant |
| Calcium hydrogen | 30.000 | Filler |
| phosphate, anhydrous Silica, colloidal,anhydrous | 4.000 | Flow regulator, disintegration accelerator |
| Magnesium stearate | 9.000 | Lubricant |
| Tablet Film | | |
| Hypromellose | 11.383 | Film former |
| Glyceryl tristearate | 1.138 | Plasticizer |
| Titanium dioxide (E 171) | 0.783 | Colouring agent |
| Talc | 3.131 | Coating agent |
| Ferric oxide, red (E 172) | 1.565 | Colouring agent |

The extract is mixed with the excipients of the tablet core and compressed on a suitable tablet press.

**Table B**

| Name of ingredient | Quantity per film coated tablet [mg / 658.000 mg] | Function |
|---|---|---|
| Tablet Core | | |
| Vitis viniferae folium dry extract aqueous (4-6:1) | 360.000 | Active ingredient |
| Silica, colloidal,anhydrous | 15.000 | binder |
| Microcrystalline cellulose | 214.000 | Binder, disintegrant |
| Croscarmellose sodium | 18.000 | Disintegrant |
| Calcium hydrogen phosphate, anhydrous | 30.000 | Filler |
| Silica, colloidal,anhydrous | 6.000 | Flow regulator, disintegration accelerator |
| Magnesium stearate | 9.000 | Lubricant |
| Crospovidone | 18.000 | Disintegrant |
| Tablet Film | | |
| Hypromellose | 11.383 | Film former |
| Glyceryl tristearate | 1.138 | Plasticizer |
| Titanium dioxide (E 171) | 0.783 | Colouring agent |
| Talc | 3.131 | Coating agent |
| Ferric oxide, red (E 172) | 1.565 | Colouring agent |

The extract is mixed with 15.000 mg silica during the drying process, which yields an extract consisting of 96 % by weight of the ingredients of the extract and 4 % of silica. This resulting mixture is mixed with the remaining excipients of the tablet core and compressed on a suitable tablet press.

The compression forces which are needed to produce tablets of suitable breaking resistance and hence with the required breakdown times are dependent on the shapes and sizes of the punching tools used. Compression forces in the range from 2 - 20 kN are preferred. Higher compression forces may lead to tablets with a delayed released of active substance. Lower compression forces may produce mechanically unstable tablets. The tablet cores may have different shapes; the preferred shapes are round biplanar or biconvex and oval or oblong forms.

The coating solution is prepared by mixing the film-forming agent with the colouring materials and a plasticizer in water. Using a suitable coating pan the film-coating solution is applied on to the tablet cores.

Preferably the tablets have an oblong shape to facilitate swallowing. In the case of a film-coated tablet containing 360 mg of extract and an extract to excipient ratio as indicated before, an oblong tablet may be about 17-18 mm long and have a width of about 8 to 9 mm. These film coated tablets of Table A are hereinbelow coded "AS 195".

To enhance the blood circulation and/or the oxygen supply of the lower extremities, the tablet should be taken in dosages corresponding to 150 and 1000 mg of extract, preferably 300-800 mg, in particular 350-750 mg daily. The total amount of extract may be divided up in 1 to 3 film coated tablets a day. The daily dose should be taken at once, preferably in the morning.

Impressive improvement of the symptoms can be expected within 6 weeks of continuous use. The optimum effect is maintained or amplified on longer use.

### Methods

### Participants

Male and female patients, age 18 years or more, with proven CVI I or CVI according to Widmer, with diagnosis confirmed and present for at least one year were enrolled. Medically relevant concomitant diseases have to be absent. Patients who used drugs to alleviate their CVI symptoms within 4 weeks or were treated with theophyllin, diuretics, cardiac glycosides, ACE inhibitors or calcium antagonists within 8 days prior to the first examination were not allowed to be enrolled. Compression bandages or concomitant therapy for venous problems were forbidden during the participation in the trial.

### Design and procedures

The double-blind, randomised, placebo-controlled cross-over trial was run according to the principles of the declaration of Helsinki and the International Conference of Harmonisation of Good Clinical Practice.

Each patient participated for 17 weeks in the trial: for a one-week wash-out (placebo-treated), for a 6-week treatment period (Group_1 starting with AS 195, Group_2 starting with placebo), for a 4-week wash-out (placebo-treated), and for a second 6-week treatment period (Group_1 continuing with placebo, Group_2 continuing with the ).
AS 195 (film-coated tablets containing 360 mg dry extract of red vine leaves) or placebo tablets were taken according to the randomisation schedule as single dose in the morning. Both tablets were identical with respect to size, shape, weight, inner appearance, and taste.
For laser Doppler flowmetry the equipment was provided by LMTB, Berlin, Germany (e.g. Doerschel K, Mueller G. Velocity resolved laser Doppler flow measurement in skin. Lasermedizin 1996;12:163-171.). The equipment is a computer-based mobile unit using a laser frequency of 785 nm. The laser probe was fixed 3.5 cm distal to the inner ankle of the more affected leg. After 30 minutes sitting for adaptation to room temperature, measurement started after 10 minutes standing (256 points of measurement, duration of measurement: approx. 0.4 seconds). The back-scattered light was retrieved by two diodes in the range of frequencies between 0.2 to 37.2 kHz. The data were processed using a Fast Fourier Transformation. Finally; the output referred to the range of frequencies between 0.2 to 10.0 kHz for vessels in the reticular venous plexus (larger mainly thermoregulative vessels, diameter more than 30 micrometer) and to the range of frequencies between 10.1 to 37.2 kHz for capillaries in the subpapillary venous plexus (superficial small nutritive vessels, diameter 6 to 30 micrometer).

Transcutaneous oxygen pressure (tcPO2) was measured using modified Clark-type polarographic electrodes containing noble metal cathodes and silver/sliver chloride anodes (TCM 3, Radiometer Copenhagen, Brønshøj, Denmark). A heating element adjacent to the anode maintained skin temperature at 43° Celsius. At this temperature the arterioles are maximally dilated, tcPO2 approximates the PO2 of arterial blood (e.g. Bollinger A, Jäger K, Jünger M, Seifert H. The vascular laboratory: advances in non-invasive techniques. World J Surg 1988;12:724-731.).

The electrode was attached to the skin surface by an adhesive ring device which was filled with physiological saline, 3.5 cm anteriolateral from the Laser Doppler probe. After 30 minutes sitting for adaptation to room temperature, measurement started after 10 minutes of standing. A measurement lasted approx. 15 minutes. The tcPO₂ values are expressed in millimeter mercury column (mmHg). Normal values available for the dorsum of the foot of patients without CVI are ranged between 40 and 80 mmHg.

Local skin temperature was measured with a thermistor fixed adjacent to the oxygen electrode in the perimalleolar region. In order to minimise effects on the skin perfusion, LDF and tcPO₂ measurements were conducted between 28 and 32° C local skin temperature.

Calf and ankle circumference were measured using a measuring tape. Measurements were carried out at the lateral and medial ankle and at the middle of the calf.

Subjective symptoms of CVI (tired heavy legs, sensation of tension, tingling sensation, and pain) were measured by using a 10-cm visual analogue scale with zero as "none at all" and 10 cm as "very strong".

Overall treatment efficacy was rated by patients and investigators on a 4-point verbal rating scale (good, satisfactory, not satisfactory, and bad) at the end of each treatment period.

Overall tolerability was rated by patients and investigators on a 4-point verbal rating scale (good, satisfactory, not satisfactory, and bad). The patients were questioned about their well-being in general terms at each visit.

### Results

Seventy-one women and men aged between 32 and 76 years with proven CVI stage I and II according to Widmer were included. The mean age (± standard deviation) was 55.2 ± 7.7 years; 55 were women, 16 men. The phlebological status revealed moderate or severe intensity of varicosis in 47 (67.1%), pigmentation in 27 (38.6%), ankle oedema in 26 (37.1%), and lower leg oedema in 25 (35.7%) patients. Mild signs of atrophy were present in 13 patients (18.6%), of eczema in none (Table 1).

**Table 1: Demographics and baseline characteristics of CVI**

| | | **AS 195 / Placebo** (n=36) | **Placebo / AS195 (n=35)** |
|---|---|---|---|
| **Continuous variates (median (range))** | | | |
| Age [years] | | 66 (32-76) | 66 (37-76) |
| Height [cm] | | 168 (150-186) | 165 (150-191) |
| Weight [kg] | | 76.5 (48-97) | 73 (55-120) |
| Body mass index [kg/m²] | | 27.6 (20.6-32.0) | 26.7 (20.1-42.5) |
| Systolic blood pressure [mmHg] | | 130 (100-150) | 135 (120-140) |
| Diastolic blood pressure [mmHg] | | 80 (60-90) | 80 (65-90) |

| **Categorical variates (n (%))** | | | |
|---|---|---|---|
| Female | | 24 (66.7) | 31 (88.6) |
| Current smoker | | 4 (11.1) | 1 (2.9) |
| CVI stage | | | |
| | Stage I | 26 (72.2) | 23 (65.7) |
| | Stage II | 10 (27.8) | 12 (34.3) |
| | Phlebological status of moderate to severe intensity | | |
| | Varicosis | 26 (72.2) | 22 (62.9) |
| | Pigmentation | 11 (30.6) | 17 (48.6) |
| | Atrophy | 0 (0.0) | 0 (0.0) |
| | Eczema | 0 (0.0) | 0 (0.0) |
| | Ankle oedema | 13 (36.1) | 14 (40.0) |
| | Lower leg oedema | 12 (33.3) | 14 (40.0) |

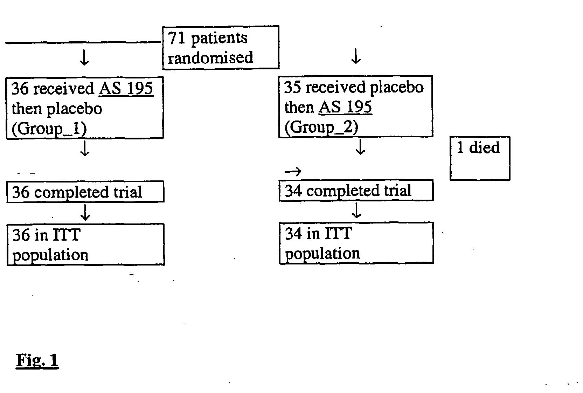

Protocol violations did not occur in the remaining patients. Therefore, all patients remained in the intention to treat analyses (Figure 1). Patient characteristics were homogenously distributed across the two treatment sequences (Group_1, Group_2) except for the sex ratio (12 men in Group_1, 4 men in Group_2) (Table 1). Baseline values for the laser Doppler parameters, transcutaneous oximetry, ankle and calf circumferences, and subjective symptoms were comparable for Group_1 and Group_2 (Table 2). Compliance with the film coated tablet according to the invention was approximately 100% in both treatment sequences.

**Table 2: Mean (±SD) of baseline characteristics of each treatment period**

| | | **Period 1** | | **Period 2** | |
|---|---|---|---|---|---|
| | | **AS 195** | **Placebo** | **AS 195** | **Placebo** |
| | | **(n=36)** | **(n=34)** | **(n=34)** | **(n=36)** |
| **Laser Doppler Flowmetry [AU]** | | | | | |
| | 10-37 kHz | 303.5 (135.2) | 333.5 (153.0) | 275.4 (126.4) | 293.3 (119.9) |
| | < 10 kHz | 352.7 (87.7) | 370.8 (120.0) | 174.7 (77.0) | 189.4 (67.6) |
| **Transcutaneous Oximetry [mmHg]** | | 32.1 (7.0) | 32.3 (6.4) | 30.1 (6.2) | 30.8 (6.4) |
| **Circumference [cm]** | | | | | |
| | Ankle | 20.3 (2.2) | 20.4 (2.4) | 20.2 (2.6) | 20.3 (2.2) |
| | Calf | 34.7 (3.1) | 34.2 (3.0) | 34.0 (3.1) | 34.6 (3.2) |
| **Subjective symptoms [cm]** | | | | | |
| | Tired/heavy legs | 4.3 (2.8) | 3.7 (2.9) | 4.6 (2.9) | 5.2 (2.6) |
| | Pain in legs | 4.0 (3.2) | 3.2 (3.1) | 4.5 (2.7) | 4.9 (3.1) |
| | Sensation of tension | 4.5 (2.9) | 4.1 (2.8) | 4.5 (2.6) | 5.1 (2.5) |
| | Tingling sensation | 3.3 (3.1) | 2.7 (2.9) | 3.7 (2.6) | 4.2 (2.8) |

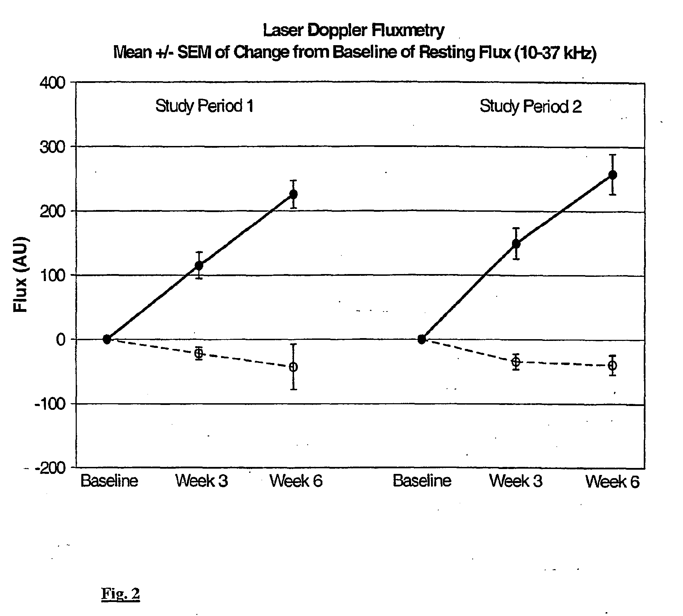

Laser Doppler Flow measurements in the frequency range of 10-37 kHz were elected for the primary endpoint. These frequencies are considered to be determined by the number of erythrocytes and their movements (flow velocity) in the capillaries of the superficial layer of the skin of the leg. After 6 weeks the laser Doppler frequencies (10-37 kHz) increased in the AS 195 group (plus 241.8±18.7 AU) but decreased in the placebo group (minus 41.0±18.7 AU, p<0.0001) (Table 3). This effect was present as early as 3 weeks after start of treatment (p<0.0001) (Table 4, Figure 2).

**Table 3: Mean (±SEM) of change from baseline adjusted for period effects, 95 % confidence interval for treatment contrasts and p value after 3 weeks treatment with 360 mg AS 195 or placebo**

| | | **Treatment** | | **Treatment contrast** | | |
|---|---|---|---|---|---|---|
| | | **AS 195 (n=70)** | **Placebo (n=70)** | **Difference (n=70)** | **Confidence interval (n=70)** | **p value** |
| **Week 3** | | | | | | |
| **Laser Doppler Flowmetry [AU]** | | | | | | |
| | 10-37 kHz | 132.2 (11.9) | -28.2 (11.9) | 160.5 | 127.0 to 194.0 | < 0.0001 |
| | < 10 kHz | -3.7 (9.2) | -99.9 (9.2) | 96.2 | 70.2 to 122.2 | < 0.0001 |
| **Transcutaneous Oximetry [mmHg]** | | 0.62 (0.97) | -3.84 (0.97) | 4.46 | 1.72 to 7.20 | 0.0018 |
| | **Circumference [cm]** | | | | | |
| | Ankle | -0.19 (0.09) | 0.21 (0.09) | -0.40 | -0.65 to -0.15 | 0.0025 |
| | Calf | -0.24 (0.04) | 0.04 (0.04) | -0.28 | -0.40 to -0.17 | < 0.0001 |
| **Subjective symptoms [cm]** | | | | | | |
| | Tired/heavy legs | -0.94 (0.25) | 0.21 (0.25) | -0.73 | -1.42 to -0.04 | 0.0396 |
| | Pain in legs | -1.17 (0.23) | - 0.24 (0.23) | -0.94 | -1.59 to -0.28 | 0.0061 |
| | Sensation of tension | -1.00 (0.24) | - 0.52 (0.24) | -0.49 | -1.17 to 0.19 | 0.1588 |
| | Tingling sensation | -0.99 (0.26) | - 0.20 (0.26) | -0.79 | -1.52 to -0.06 | 0.0335 |

**Table 4: Mean (±SEM) of change from baseline adjusted for period effects, 95 % confidence interval for treatment contrasts and p value after 6 weeks treatment with 360 mg AS 195 or placebo**

| | | **Treatment** | | **Treatment contrast** | | |
|---|---|---|---|---|---|---|
| | | **AS 195 (n=70)** | **Placebo (n=70)** | **Difference (n=70)** | **Confidence interval (n=70)** | **p value** |
| **Week 6** | | | | | | |
| **Laser Doppler Flowmetry [AU]** | | | | | | |
| | 10-37 kHz **(primary endpoint)** | 241.8 (18.7) | -41.0 (18.7) | 282.8 | 229.9 to 335.7 | < 0.0001 |
| | < 10 kHz | 57.0 (12.4) | -107.7 (12.4) | 164.7 | 129.7 to 199.7 | < 0.0001 |
| **Transcutaneous Oximetry [mmHg]** | | 1.35 (0.97) | -7.27 (0.97) | 8.63 | 5.88 to 11.38 | < 0.0001 |
| | **Circumference [cm]** | | | | | |
| | Ankle | -0.39 (0.09) | 0.29 (0.09) | -0.68 | -0.94 to -0.43 | < 0.0001 |
| | Calf | -0.54 (0.05) | 0.14 (0.05) | -0.68 | -0.83 to -0.53 | < 0.0001 |
| **Subjective symptoms [cm]** | | | | | | |
| | Tired/heavy legs | -0.78 (0.33) | -0.94 (0.33) | 0.16 | -0.76 to 1.09 | 0.7285 |
| | Pain in legs | -0.76 (0.35) | -0.86 (0.35) | 0.10 | -0.88 to 1.09 | 0.8323 |
| | Sensation of tension | -0.96 (0.35) | - 1.40 (0.35) | 0.44 | -0.46 to 1.44 | 0.3819 |
| | Tingling sensation | -0.55 (0.30) | - 0.66 (0.30) | 0.11 | -0.75 to 0.96 | 0.8044 |

Laser Doppler Flow measurements in the frequency range below 10 kHz are considered to be determined by the number of erythrocytes and their movements (flow velocity) in the capillaries in the deeper mainly thermoregulative layer of the skin of the leg. After 6 weeks the laser Doppler frequencies below 10 kHz) increased in the AS 195 group (plus 57.0±12.4 AU) and decreased in the placebo group (minus 107.7±12.4 AU, p<0.0001) (Table 3). This effect seems to depend on the climatic condition during the treatment period. During the study period of moderate temperatures (April/May) the Laser Doppler measurements (<10 kHz) remained unchanged in the AS 195 treatment group after an initial drop whereas the measurements in the placebo group decreased (p<0.0001). During the study period of higher temperatures (July/August) the laser Doppler measurements (<10 kHz) increased in the AS 195 treatment group and remained constant in the placebo group. (p<0.0001).

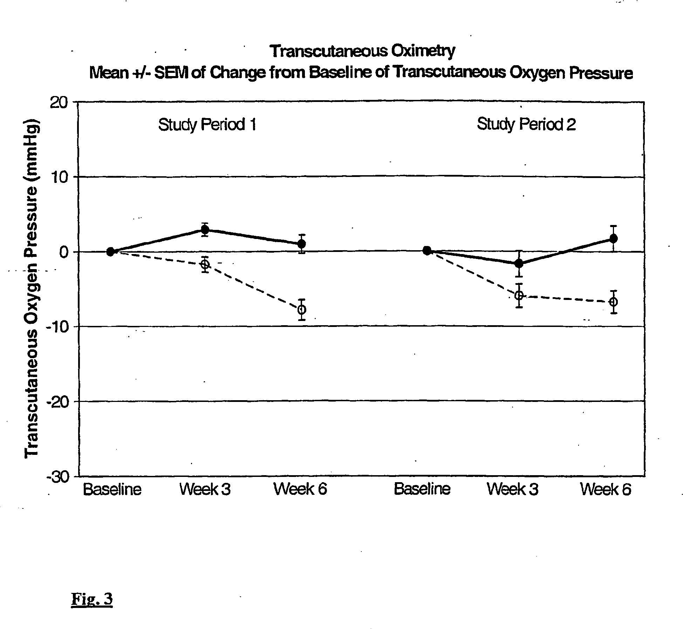

The transcutaneous oxygen pressure increased in the AS 195 group (plus 1.35±0.97 mmHg) but decreased in the placebo group (minus 7.27±0.97 mmHg, p<0.0001). This observation was consistent in both treatment periods and would therefore be in line with the Laser Doppler Flow in the nutritive superficial layer of the skin (i.e.,10-37 kHz) (Table 3,4, Figure 3).

The statistically significant and clinically relevant reduction of ankle (after 3 weeks: AS 195 minus 0.19±0.09 cm, placebo plus 0.21±0.09 cm, p=0.0025) and calf circumferences (after 3 weeks: AS 195 minus 0.24±0.04 cm, placebo plus 0.04±0.04 cm, p<0.0001) indicate an onset of action as early as 3 weeks after start of treatment (Table 3). This effect becomes more pronounced after 6 weeks (AS 195 ankle: minus 0.39±0.09 cm, calf: minus 0.54±0.05; placebo ankle: plus 0.29±0.09 cm, calf: plus 0.14±0.05 cm, p<0.0001) (Table 4)

There was no relevant change of the intensity of the subjective symptoms related to CVI after 6 weeks of treatment. This result is in line with those of a previous study where subjective symptoms measured on a visual analogue scale were reduced only after longer treatment periods (12 weeks).

Adverse events occurred rarely in this study. Thirteen of 71 patients experienced at least one adverse event, 12 of them experienced the onset of action while on placebo treatment, one while on AS 195 (bronchitis, moderate intensity, considered not drug related by the investigator). The patient who died from cardiac arrest had been treated with placebo (never received AS 195 in this trial). All patients assessed the overall tolerability as good or satisfactory. The laboratory parameters did not change during the study.

### Discussion

It has been shown in a previous study (WO 01/28363) that red vine leaves extract AS 195 reduces lower leg oedema, calf circumference, and ankle circumference in addition to improving subjective symptoms related to chronic venous insufficiency in patients treated once daily for 12 weeks.⁷ The present study was designed to provide additional information on the underlying mechanism of action by investigating microcirculation as a clinically relevant surrogate parameter for CVI related leg problems. This study is the first one in CVI patients aimed to investigate in addition to leg oedema reduction further clinical relevant effects related to the therapy with red vine leaves extract. The reduced venous drainage results in impaired cutaneous microcirculation with trophical disturbances of the skin. If CVI remains untreated this condition may even result venous leg ulcers. Laser Doppler flowmetry, as used in the present study, is a valid and sensitive method to measure objective treatment effects which may be related to the subjectively experienced volume reduction after 3 months of treatment.

The study results fit into the clinical data available for AS 195 and add information on the onset of action: The leg volume as an objective parameter will be reduced in a clinically relevant and statistically significant degree after 6 weeks of treatment. This objective effect has also been reported recently with horse chestnut sees extract (e.g. Diehm C, Trampisch HJ, Lange S, Schmidt C. Comparison of leg compression stocking and oral horse-chestnut seed extract therapy in patients with chronic venous insufficiency. Lancet 1996;347:292-294.) and Butchers Broom (e.g. Vanscheidt W, Jost V, Wolna P, et al. Efficacy and safety of a Butcher's Broom preparation (Ruscus aculeatus L. extract) compared to placebo in patients suffering from chronic venous insufficiency. Drug Res 2002;52(4):243-250.).

In the present study it was shown that the laser Doppler flowmetry parameters, the ankle and calf circumferences and the transcutaneous oxygen pressure were affected as early as after 3 weeks of treatment. In contrast, the subjective symptoms of CVI rated on a visual analogue scale were not significantly different from placebo after 6 weeks of treatment as they were in the previous study. A treatment duration of 12 weeks is mandatory for a relevant reduction of subjective CVI symptoms.

The present results suggest a major role of red vine leaves extract in prevention of CVI progression and the occurrence of trophical skin lesions and may even prevent or delay the transition from clinically not relevant early stages of CVI to CVI Stage I.

## Claims

1. A film coated tablet having enhanced stability comprising:
(a) 50 to 70% by weight of a dried aqueous extract of red vine leaves;
(b) 25 % to 49 % by weight of a excipient; and
(c) 1 % to 5 % by weight of a tablet film;
based on the total mass of the film coated tablet,
wherein the dried aqueous extract of red vine leaves is obtainable by a drying process comprising the step of adding silica during the drying process.

2. The film coated tablet of claim 1, wherein said dried aqueous extract of vine leaves is obtainable by a method comprising the steps of:
(a) collecting red vine leaves at a point of time when the content in flavonoids has reached an optimum;
(b) drying and crushing the leaves;
(c) cutting the leaves to pieces;
(d) extracting the leaves with water at elevated temperatures for 6 to 10 hours;
(e) concentrating and drying the obtained extract;
(f) addition of up to 10 % by weight of silica relating to the final total amount of the resulting extract.

3. The film coated tablet of claim 2, wherein the silica is colloidal anhydrous silica.

4. The film coated tablet of claim 3, wherein said extract comprises 2.5 to 7.5 % by weight of colloidal, anhydrous silica.

5. The film coated tablet of claim 4, wherein said extract comprises about 4.0 % by weight of colloidal, anhydrous silica.

6. The film coated tablet of claim 1, wherein the excipient comprises a binder in an amount by weight based on the total mass of the tablet from 15 to 45%, preferably 25 to 40%, and
wherein the binder is selected among powdered cellulose, microcrystalline cellulose, starch, polyvinylpyrrolidone, copolymers of vinylpyrrolidone with other vinyl derivatives, cellulose derivatives or a mixture thereof.

7. A film coated tablet of claim 1, wherein the tablet film (c) essentially consists of 50 to 85 % by weight of at least one film former, 5 to 10 % by weight of at least one plasticizer, 10 to 20 % by weight of at least one coating agent, and 0 to 15 % by weight of at least one colorant, based on the total mass of the tablet film (c).

## Patentansprüche

1. Filmtablette mit verbesserter Stabilität, umfassend:
(a) 50 bis 70 Gew.-% eines getrockneten wässerigen Extrakts von roten Weinblättern;
(b) 25 bis 40 Gew.-% eines Hilfsstoffs und
(c) 1 bis 5 Gew.-% eines Tablettenfilms;
basierend auf der Gesamtmasse der Filmtablette,
wobei der getrocknete wässerige Extrakt von roten Weinblättern erhältlich ist durch ein Trocknungsverfahren, umfassend den Schritt der Zugabe von Siliciumoxid während des Trocknungsverfahrens.

2. Filmtablette nach Anspruch 1, wobei der getrocknete wässerige Extrakt von Weinblättern erhältlich ist durch ein Verfahren, umfassend die Schritte:
(a) Sammeln von roten Weinblättern zu einem Zeitpunkt, wo der Gehalt an Flavonoiden sein Maximum erreicht hat;
(b) Trocknen und Zerkleinern der Blätter;
(c) Schneiden der Blätter in Stückchen;
(d) Extrahieren der Blätter mit Wasser bei erhöhter Temperatur für 6 bis 10 Stunden;
(e) Konzentrieren und Trocknen des erhaltenen Extrakts;
(f) Zugabe von bis zu 10 Gew.-% Siliciumoxid zur endgültigen Gesamtmenge des resultierenden Extrakts.

3. Filmtablette nach Anspruch 2, wobei das Siliciumoxid kolloidales wasserfreies Siliciumoxid darstellt.

4. Filmtablette nach Anspruch 3, wobei der Extrakt 2,5 bis 7,5 Gew.% kolloidales wasserfreies Siliciumoxid umfasst.

5. Filmtablette nach Anspruch 4, wobei der Extrakt etwa 4,0 Gew.-% kolloidales wasserfreies Siliciumoxid umfasst.

6. Filmtablette nach Anspruch 1, wobei der Hilfsstoff ein Bindemittel in einer Gew.-Menge, bezogen auf die Gesamtmasse der Tablette, von 15 bis 45%, bevorzugt 25 bis 40% umfasst, und worin das Bindemittel ausgewählt ist aus pulverisierter Cellulose, mikrokristalliner Cellulose, Stärke, Polyvinylpyrrolidon, Copolymeren von Vinylpyrrolidon mit anderen Vinylderivaten, Cellulosederivaten oder einer Mischung davon.

7. Filmtablette nach Anspruch 1, wobei der Tablettenfilm (c) im Wesentlichen besteht aus 50 bis 85 Gew.-% von mindestens einem Filmbildner, 5 bis 10 Gew.-% von mindestens einem Weichmacher, 10 bis 20 Gew.-% von mindestens einem Beschichtungsmittel und 0 bis 15 Gew.-% von mindestens einem farbgebenden Mittel, basierend auf der Gesamtmasse des Tablettenfilms (c).

## Revendications

1. Comprimé enrobé avec un film ayant une stabilité accrue comprenant :
(a) 50 à 70 % en poids d'un extrait aqueux séché de feuilles de vigne rouge ;
(b) 25 % à 49 % en poids d'un excipient ; et
(c) 1 % à 5 % en poids d'un film pour comprimé ;
sur la base de la masse totale du comprimé enrobé avec un film,
où l'extrait aqueux séché de feuilles de vigne rouge peut être obtenu par un procédé de séchage comprenant l'étape d'addition de silice pendant le procédé de séchage.

2. Comprimé enrobé avec un film selon la revendication 1 où ledit extrait aqueux séché de feuilles de vigne peut être obtenu par un procédé comprenant les étapes de :
(a) recueillir des feuilles de vigne rouge à un moment où la teneur en flavonoïdes a atteint un optimum ;
(b) sécher et broyer les feuilles ;
(c) couper les feuilles en fragments ;
(d) extraire les feuilles avec de l'eau à des températures élevées pendant 6 à 10 heures ;
(e) concentrer et sécher l'extrait obtenu ;
(f) ajouter jusqu'à 10 % en poids de silice par rapport à la quantité totale finale de l'extrait résultant.

3. Comprimé enrobé avec un film selon la revendication 2 où la silice est de la silice anhydre colloïdale.

4. Comprimé enrobé avec un film selon la revendication 3 où ledit extrait comprend 2,5 à 7,5 % en poids de silice anhydre colloïdale.

5. Comprimé enrobé avec un film selon la revendication 4 où ledit extrait comprend environ 4,0 % en poids de silice anhydre colloïdale.

6. Comprimé enrobé avec un film selon la revendication 1 où l'excipient comprend un liant en une quantité en poids sur la base de la masse totale du comprimé de 15 à 45 %, de préférence de 25 à 40 %, et où le liant est choisi parmi la cellulose pulvérulente, la cellulose microcristalline, l'amidon, la polyvinylpyrrolidone, les copolymères de vinylpyrrolidone et d'autres dérivés vinyliques, les dérivés cellulosiques ou un mélange de ceux-ci.

7. Comprimé enrobé avec un film selon la revendication 1 où le film pour comprimé (c) consiste essentiellement en 50 à 85 % en poids d'au moins un agent filmogène, 5 à 10 % en poids d'au moins un plastifient, 10 à 20 % en poids d'au moins un agent d'enrobage et 0 à 15 % en poids d'au moins un colorant, sur la base de la masse totale du film pour comprimé (c).
